Europäisches Patentamt

European Patent Office

Office européen des brevets

(11) Numéro de publication : **0 346 185 B1**

(19)

(12) # FASCICULE DE BREVET EUROPEEN

(45) Date de publication du fascicule du brevet :
30.12.92 Bulletin 92/53

(51) Int. Cl.⁵ : **C07C 205/02,** C07C 201/10

(21) Numéro de dépôt : **89401485.1**

(22) Date de dépôt : **31.05.89**

(54) **Procédé de fabrication de nitrométhane.**

(30) Priorité : **09.06.88 FR 8807675**

(43) Date de publication de la demande :
**13.12.89 Bulletin 89/50**

(45) Mention de la délivrance du brevet :
**30.12.92 Bulletin 92/53**

(84) Etats contractants désignés :
**BE DE ES FR GB IT LU NL SE**

(56) Documents cités :
**EP-A- 0 146 203**
**EP-A- 0 146 204**
**US-A- 4 469 904**

(56) Documents cités :
**JOURNAL OF THE AMERICAN CHEMICAL
SOCIETY, vol. 76, no. 10, 26 mai 1954, pages
2601,2692-2694, The American Chemical
Society;H.B. HASS et al.: "Vapor phase nitration of aliphatic ethers, alcohols, ketones and
carboxylic acids"**

(73) Titulaire : **W.R. Grace & Co.-Conn.
1114 Avenue of the Americas
New York New York 10036 (US)**

(72) Inventeur : **Mari, Roger
8, avenue de Brabois
F-54600 Villers-les-Nancy (FR)**
Inventeur : **Quibel, Jacques
40, rue de la Muette
F-78600 Maisons Laffite (FR)**

(74) Mandataire : **Durand, Yves Armand Louis et al
Cabinet Z. Weinstein 20, Avenue de Friedland
F-75008 Paris (FR)**

EP 0 346 185 B1

## Description

La présente invention concerne la préparation de nitrométhane.

De nombreux procédés ont été proposés pour fabriquer du nitrométhane, notamment par nitration directe du méthane. Cependant il est bien connu que le méthane est beaucoup plus mal aisé à nitrer que les hydrorarbures plus lourds, en raison de sa plus grande stabilité. Souvent, les taux de conversion du méthane en nitrométhane sont restés faibles (voir par exemple documents EP-A-0 146 204 et EP-A-0 146 203).

Aussi, on a recherché, à partir de matières premières courantes de la grande industrie chimique, un procédé de production de nitrométhane diversifié par rapport au méthane. Selon l'invention, il est proposé un procédé utilisant comme matières premières d'une part un agent nitrant formé de composés oxygénés de l'azote et d'autre part un composé oxocarboné comportant deux atomes de carbone et au moins un atome d'oxygène, la réaction étant mise en oeuvre en présence de dichloroéthane comme catalyseur.

Il a été constaté que les composés oxocarbonés comportant deux atomes de carbone et au moins un atome d'oxygène conduisent à des rendements appréciables en nitrométhane par rapport au composé oxocarboné.

Le composé oxocarboné peut être avantageusement choisi parmi l'éthanol, l'acide acétique et l'acétaldéhyde.

L'agent nitrant est choisi parmi les composés oxygénés de l'azote tels, que l'acide nitrique et le bioxyde d'azote $NO_2$ utilisés seuls ou en mélange. L'acide nitrique peut avoir une concentration comprise entre 50 et 100% et plus avantageusement la concentration commerciale.

En outre, en présence du catalyseur, les rendements en nitrométhane par rapport au composé oxocarboné sont supérieurs à ceux obtenus en l'absence du catalyseur. La présence du dichloroéthane est donc très importante, notamment lorsque dans la réaction il est mis en oeuvre en proportion molaire de l'ordre de 0,2 à 1% par rapport au composé oxocarboné à deux atomes de carbone.

Le choix du rapport molaire composé oxocarboné/agent nitrant influe sur les résultats.

Quand le rapport entre le nombre de molécules oxocarbonées et le nombre de molécules nitrantes est inférieur à 1, le procédé conduit à l'obtention d'un mélange riche en nitrométhane. Dans le cas, où l'agent oxocarboné est l'éthanol on obtient un mélange riche en nitrométhane, contenant une quantité importante d'acide acétique que l'on transforme en nitrométhane dans une phase ultérieure. De préférence, ce rapport molaire est compris entre 0,2 et 0,9.

Quand le rapport entre le nombre de molécules oxocarbonées et le nombre de molécules de l'agent nitrant est supérieur à 1, de préférence compris entre 1,2 et 1,5, le nitrométhane apparait avec le composé carboné n'ayant pas réagi, et le dit composé est recyclé en vue d'une opération identique ultérieure.

La pression de réaction nitrante est maintenue entre 1 et 30 bars, les températures de réaction sont comprises entre 220°C et 500°C, de préférence entre 280° et 430°C. Quand la matière première oxocarbonée est l'éthanol, la température réactionnelle est favorablement comprise entre 290°C et 330°C, et lorsque l'agent oxocarboné est l'acide acétique cette plage de températures se situe plutôt entre 360 et 430°C.

Le temps de contact réactionnel est fonction de la température, de la pression et de la composition du mélange pour obtenir le résultat attendu, par exemple un temps de réaction de 10 à 12 secondes pour la réaction à partir d'éthanol, à 325°C, avec un rapport éthanol/agent nitrant de l'ordre de 1,3 conduit à un rendement de l'ordre de 60% en moles de nitrométhane par rapport à l'alcool consommé.

Pour obtenir des résultats équivalents, il convient d'ajuster le temps de réaction en fonction de la température. Par exemple, le temps de réaction optimum se trouvant de 10,2 secondes, pour une pression de 3 bars, à 325°C, avec un rapport éthanol/acide nitrique de 1,32, un taux de catalyseur (dichloréthane) de 0,3%, conduisant à un rendement de 64,4% pour un taux de conversion de 13,4, la température passant à 340°C, ce temps optimum descend à 8 secondes et monte à 12,5 secondes à 310°C. Néanmoins, dans ces conditions, le rendement le plus élevé est obtenu à 325°C.

A conditions réactionnelles identiques, la présence de catalyseur améliore le rendement et le taux de conversion. Par exemple à 320°C, sous une pression de 3 bars, un rapport éthane/acide nitrique de 1,15, on obtient en présence de 0,5% de catalyseur (dichloréthane) un rendement de 31,6% et un taux de conversion de 15,6%, alors qu'en absence de catalyseur ces résultats chiffrés sont respectivement de 18,8% et 11,2%. Toutefois, dans ce dernier cas, on note l'apparition d'acétaldéhyde à raison de 4% et d'acide acétique à raison de 1%, matières premières convertissables. En présence de 0,3% de catalyseur, les résultats sont sensiblement identiques à ceux obtenus en présence de 0,54%.

En vue de modifier le temps de réaction, le mélange réactionnel peut être dilué par un agent diluant inerte vis-à-vis de réactifs et des produits formés dans la réaction. L'agent diluant inerte peut être choisi parmi l'azote ou l'eau, introduit en quantité fonction de la modification attendue.

Il est donné ci-après des exemples qui illustrent l'invention à titre non limitatif.

## Exemple 1

A une température de 310°C, sous une pression de 6 bars, on met en réaction de l'acide nitrique, à titre d'agent nitrant et de l'éthanol composé carboné oxygéné, dans un rapport molaire éthanol/acide nitrique de 0,23, en présence de 0,3% en moles de dichloréthane par rapport à l'éthanol. Le temps de séjour réactionnel est de 9,2 secondes.

On obtient, pour 100 moles d'éthanol, 49 moles de nitrométhane et 55 moles d'acide acétique, le reste étant du carbone apparaissant sous forme de dioxyde de carbone $CO_2$ et de monoxyde de carbone CO avec prédominance de CO. Cet exemple illustre un rapport entre le nombre de molécules carbonées et le nombre de molécules nitrantes inférieur à 1, et dans lequel l'agent carboné : l'éthanol donne un mélange riche en nitrométhane, contenant une quantité importante d'acide acétique transformé dans une phase ultérieure en nitrométhane.

## Exemple 2

A 320°C sous une pression de 3 bars, on met en réaction de l'acide nitrique et de l'éthanol dans un rapport molaire éthanol/acide nitrique de 1,32, avec un temps de séjour de 10,2 secondes, en présence de 0,3% moles de dichloroéthane par rapport à l'éthanol.

On obtient pour 100 moles d'éthanol, 13,4 moles de nitrométhane, on retrouve 79 moles d'éthanol, le reste du carbone apparaissant sous forme de $CO_2$ et de CO avec prédominance de CO. Cet exemple illustre un rapport entre le nombre de molécules nitrantes, supérieur à 1, et dans lequel le nitrométhane apparait avec l'éthanol restant.

L'éthanol n'ayant pas réagi est recyclé dans une opération identique ultérieure.

## Exemple 3

A une température de 410°C, sous une pression de 6 bars, pendant un temps de séjour de 10,5 secondes, on fait réagir l'acide nitrique et l'acide acétique dans un rapport molaire acide acétique/acide nitrique de 1,2, en présence de 0,3% moles de dichloréthane par rapport à l'acide acétique.

On obtient pour 100 moles d'acide acétique, 14 moles de nitrométhane, on retrouve 75 moles d'acide acétique, le reste du carbone apparaissant sous forme de dioxyde de carbone et de monoxyde de carbone, avec prédominance de CO. Cet exemple illustre un rapport molaire acide acétique/agent nitrant supérieur à 1, et dans lequel le nitrométhane apparait avec l'acide acétique n'ayant pas réagi qui est recyclé dans une opération identique ultérieure.

## Exemple 4

A une température de 390°C, sous une pression de 6 bars, on fait réagir l'acide acétique et l'acide nitrique dans un rapport molaire acide acétique/acide nitrique de 0,24, pendant un temps de séjour de 9,3 secondes, en présence de 0,3% moles de dichloroéthane par rapport à l'acide acétique.

On obtient pour 100 moles d'acide acétique, 20 moles de nitrométhane, on retrouve 50 moles d'acide acétique, le reste du carbone apparaissant sous forme de $CO_2$ et CO avec prédominance de CO. Cet exemple illustre un rapport molaire acide acétique/agent nitrant inférieur à 1, et dans lequel le nitrométhane apparait en grande proportion.

## Exemple 5

A une température de 335°C, sous une pression de 6 bars, on fait réagir l'acétaldéhyde et l'acide nitrique dans un rapport molaire acétaldéhyde/acide nitrique de 0,4, en présence de 0,3% moles de dichloréthane par rapport à l'acétaldéhyde.

Avec un temps de séjour de 10 secondes, on obtient pour 100 moles d'acétaldéhyde, 30 moles de nitrométhane, on retrouve 14 moles d'acide acétique, le reste du carbone apparaissant sous forme de $CO_2$ et CO avec prédominance de CO. Cet exemple illustre un rapport acétaldéhyde/agent nitrant inférieur à 1, et dans lequel le nitrométhane apparait en grande proportion.

## Exemple 6

Dans cet exemple, on procédé à l'étude de l'influence de la température, avec un rapport composé car-

3

boné/agent nitrant supérieur à 1 une pression de 3 bars, sur la réaction de l'acide nitrique et de l'éthanol, le rapport molaire éthanol/acide nitrique étant de 1,3, en présence de 0,3% moles de dichloréthane par rapport à l'éthanol, avec un temps de séjour compris entre 9,5 et 10,3 secondes.

On fait évoluer la température de 270 à 380°C, et les résultats obtenus exprimés en rendement molaire et éthanol transformé sont consignés dans le tableau I suivant :

TABLEAU I

| Température | Rendement molaire nitrométhane / éthanol | Nombre de moles d'éthanol transformées |
|---|---|---|
| 270°C | 9,7 % | 6,9 % |
| 315°C | 48,8 % | 13,3 % |
| 325°C | 64,4 % | 13,4 % |
| 340°C | 58,9 % | 12,9 % |
| 365°C | 49,1 % | 12,2 % |
| 380°C | 7,5 % | 3,5 % |

Exemple 7

Dans cet exemple on procède au même type d'étude que précédemment, avec un rapport molaire composé carboné/agent nitrant inférieur à 1.

Sous une pression de 6 bars, on fait réagir de l'acide nitrique et de l'acide acétique dans un rapport molaire de 0,3 en présence de 0,3% en moles de dichloréthane par rapport à l'acide acétique, avec un temps de séjour compris entre 9,5 et 10,5 secondes.

L'influence de la température par rapport à l'acide acétique, avec un temps de séjour compris entre 9,5 et 10,5 secondes.

L'influence de la température par rapport au rendement en nitrométhane et à l'acide acétique transformé apparait dans le tableau II ci-après :

TABLEAU II

| Température | Rendement molaire nitrométhane/acide acétique | Nombre de moles $CH_3$ CO OH transformées |
|---|---|---|
| 365°C | 97,7 % | 8,4 % |
| 375°C | 60,4 % | 14,2 % |
| 385°C | 41,9 % | 20,3 % |
| 395°C | 20,3 % | 19,4 % |
| 405°C | 18,3 % | 20,0 % |
| 415°C | 11,6 % | 11,1% |

Exemple 8

Etude de l'influence de la pression.

La température étant de 310°C, l'agent nitrant l'acide nitrique, l'agent carboné l'éthanol, le rapport molaire éthanol/acide nitrique de 0,87, le catalyseur (0,3% mole/éthanol) le dichloréthane, le temps de séjour de 10 secondes la pression agit comme indiqué dans le tableau III :

TABLEAU III

| | Pressions | | Rendement molaire Nitrométhane/éthanol | | Nombre de moles de $C_2H_5OH$ transformées | |
|---|---|---|---|---|---|---|
| : | | : | | : | | : |
| : | 3 bars | : | 11,3 % | : | 10,7 % | : |
| : | 5 bars | : | 18,3 % | : | 13,0 % | : |

**Revendications**

1. Procédé de préparation de nitrométhane dans lequel intervient un agent nitrant constitué par un composé oxygéné de l'azote, caractérisé en ce que ledit agent nitrant est mis en réaction avec un composé oxocarboné comportant deux atomes de carbone et au moins un atome d'oxygène, la réaction étant mise en oeuvre en présence de dichloroéthane comme catalyseur.

2. Procédé de préparation de nitrométhane selon la revendication 1, caractérisé en ce que le composé oxocarboné est choisi parmi l'éthanol, l'acide acétique et l'acétaldéhyde.

3. Procédé de préparation de nitrométhane selon la revendication 1 ou 2, caractérisé en ce que l'agent nitrant est l'acide nitrique ou le bioxyde d'azote utilisés seuls ou en mélange.

4. Procédé de préparation de nitrométhane selon l'une quelconque des revendications 1 à 3, caractérisé en ce que le catalyseur de dichloroéthane est mis en oeuvre en proportion molaire de l'ordre de 0,2 à 1% par rapport au composé oxocarboné à deux atomes de carbone.

5. Procédé de préparation de nitrométhane selon l'une quelconque des revendications 1 à 4, caractérisé en ce que la température réactionnelle est comprise entre 200 et 500°C, de préférence entre 280° et 430°C, la pression réactionnelle étant comprise entre 1 et 30 bars.

6. Procédé de préparation de nitrométhane selon l'une quelconque des revendications 1 à 5, caractérisé en ce que le rapport molaire composé oxocarboné/agent nitrant est inférieur à 1, de préférence compris entre 0,2 et 0,9.

7. Procédé de préparation de nitrométhane selon l'une quelconque des revendications 1 à 5, caractérisé en ce que le rapport molaire composé oxocarboné/agent nitrant est supérieur à 1, de préférence compris entre 1,2 et 1,5 et la matière première oxocarbonée restante est recyclée.

8. Procédé de préparation de nitrométhane selon l'une quelconque des revendications 1 à 7, caractérisé en ce que le mélange réactionnel est dilué par un agent diluant inerte vis-à-vis des réactifs et des produits de la réaction.

**Patentansprüche**

1. Verfahren zur Vorbereitung von Nitromethan, bei welchem ein durch eine sauerstoffhaltige Stickstoffverbindung gebildetes Nitriermittel eingreift, dadurch gekennzeichnet, dass man das besagte Nitriermittel mit einer zwei Kohlenstoffatome und wenigstens ein Sauerstoffatom aufweisenden oxokohlenstoffhaltigen Verbindung reagiren lässt, wobei die Reaktion in Anwesenheit von Dichloroäthan als Katalysator durchgeführt wird.

2. Verfahren zur Vorbereitung von Nitromethan gemäss Anspruch 1, dadurch gekennzeichnet, dass die oxokohlenstoffhaltige Verbindung unter Athanol, Essigsäure und Acetaldehyd gewählt wird.

3. Verfahren zur Vorbereitung von Nitromethan gemäss Anspruch 1 oder 2, dadurch gekennzeichnet, dass das Nitriermittel Salpetersäure oder Stickstoffdioxid ist, die allein oder im Gemisch verwendet werden.

4. Verfahren zur Vorbereitung von Nitromethan nach irgendeinem der Ansprüche 1 bis 3, dadurch gekennzeichnet, dass der Katalysator aus Dichloroäthan in einem molaren Verhältnis von 0,2 bis 1 % in Beziehung auf die oxokohlenstoffhaltige Verbindung mit zwei Kohlenstoffatomen verwendet wird.

5. Verfahren zur Vorbereitung von Nitromethan nach irgendeinem der Ansprüche 1 bis 4, dadurch gekennzeichnet, dass die Reaktionstemperatur zwischen 200 und 500°C, vorzugsweise zwischen 280 und 430°C liegt, wobei der Reaktionsdruck zwischen 1 und 30 Bar liegt.

6. Verfahren zur Vorbereitung von Nitromethan nach irgendeinem der Ansprüche 1 bis 5, dadurch gekennzeichnet, dass das Molverhältnis oxokohlenstoffhaltige Verbindung/Nitriermittel kleiner als 1 ist und vorzugsweise zwischen 0,2 und 0,9 liegt.

7. Verfahren zur Vorbereitung von Nitromethan nach irgendeinem der Ansprüche 1 bis 5, dadurch gekennzeichnet, dass das Molverhältnis oxokohlenstoffhaltige Verbindung/Nitriermittel grösser als 1 ist und vorzugsweise zwischen 1,2 und 1,5 liegt und der restliche oxokohlenstoffhaltige Rohstoff zurückgeführt wird.

8. Verfahren zur Vorbereitung von Nitromethan nach irgendeinem der Ansprüche 1 bis 7, dadurch gekennzeichnet, dass das Reaktionsgemisch mit einem gegenüber den Reagenzen und den Reaktionserzeugnissen inerten Verdünnungsmittel verdünnt wird.

## Claims

1. Method of preparation of nitromethane in which is acting a nitriding agent constituted by an oxygenated compound of nitrogen, characterized in that the said nitriding agent is reacted with an oxocarbonaceous compound comprising two carbon atoms and at least one oxygen atom, the reaction being carried out in the presence of dichloroethane as a catalyst.

2. Method of preparation of nitromethane according to claim 1, characterized in that the oxocarbonaceous compound is selected among the ethanol, the acetic acid and the acetaldehyde.

3. Method of preparation of nitromethane according to claim 1 or 2, characterized in that the nitriding agent is nitric acid or nitrogen dioxide used alone or in a mixture.

4. Method of preparation of nitromethane according to any one of claims 1 to 3, characterized in that the dichloroethane catalyst is used in a molar proportion of the order of 0.2 to 1% with respect to the oxocarbonaceous compound with two atoms of carbon.

5. Method of preparation of nitromethane according to any one of claims 1 to 4, characterized in that the reaction temperature is lying between 200 and 500°C, preferably between 280 and 430°C, the reaction pressure lying between 1 and 30 bars.

6. Method of preparation of nitromethane according to any one of claims 1 to 5, characterized in that the oxocarbonaceous compound/nitriding agent molar ratio is lower than 1, preferably lying between 0.2 and 0.9.

7. Method of preparation of nitromethane according to any one of claims 1 to 5, characterized in that the oxocarbonaceous compound/nitriding agent molar ratio is higher than 1, preferably lying between 1.2 and 1.5 and the residual oxocarbonaceous stock is recycled.

8. Method of preparation of nitromethane according to any one of claims 1 to 7, characterized in that the reaction mixture is diluted with a diluting agent which is inert with respect to the reagents and to the products of the reaction.